(19) Europäisches Patentamt / European Patent Office / Office européen des brevets

(11) **EP 3 818 974 A1**

(12) **EUROPÄISCHE PATENTANMELDUNG**

(43) Veröffentlichungstag:
**12.05.2021   Patentblatt 2021/19**

(51) Int Cl.:
**A61K 9/00** (2006.01)   **A61K 9/14** (2006.01)
**A61K 9/16** (2006.01)   **A61K 47/38** (2006.01)

(21) Anmeldenummer: **19208352.5**

(22) Anmeldetag: **11.11.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH**
**45128 Essen (DE)**

(72) Erfinder:
• **DÜRR, Georg**
**53840 Troisdorf (DE)**

• **SCHLEGEL-KACHEL, Sibylle**
**79189 Bad Krozingen (DE)**
• **TSCHERNJAEW, Juri**
**63743 Aschaffenburg (DE)**
• **ÖHRLEIN, Johannes**
**40476 Düsseldorf (DE)**
• **SCHMIED, Fabian-Pascal**
**64285 Darmstadt (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(54) **FESTE PARTIKEL ENTHALTEND FESTE PRIMÄRPARTIKEL AUS IM WESENTLICHEN NATIVER CELLULOSE**

(57) Gegenstand der Erfindung sind feste Partikel enthaltend feste Primärpartikel aus im Wesentlichen nativer Cellulose und gegebenenfalls einen Binder, deren Herstellung und Verwendung.

EP 3 818 974 A1

**Beschreibung**

Gebiet der Erfindung

Gegenstand der Erfindung sind feste Partikel enthaltend feste Primärpartikel aus im Wesentlichen nativer Cellulose und gegebenenfalls einen Binder, deren Herstellung und Verwendung.

Stand der Technik

DE2921931 offenbart ein Verfahren zur Herstellung von rieselfähigen Produkten auf der Basis von Cellulosepulver, die für den Einsatz in der Pharmazie, der chemischen Industrie und der Nahrungsmittelwirtschaft geeignet sind.
Aufgabe der Erfindung war es, feste Partikel bereitzustellen, die insbesondere in Nahrungsmitteln, kosmetischen und/oder pharmazeutischen Produkten eingesetzt werden können, welche Wirkstoffe, wie Aromen, Arzneimittel etc. gut absorbieren und im Wässrigen wieder freigeben.

Beschreibung der Erfindung

Überraschenderweise wurde gefunden, dass die in Anspruch 1 beschriebenen Partikel sich vorteilhaft in Nahrungsmitteln, kosmetischen und/oder pharmazeutischen Produkten einsetzen lassen.
Gegenstand der vorliegenden Erfindung sind daher feste Partikel mit einer mittleren Partikelgröße von 15 $\mu$m bis 2000 $\mu$m enthaltend

A) feste Primärpartikel mit einer mittleren Partikelgröße von 3 $\mu$m bis 20 $\mu$m, welche zu mindestens 95 Gew.-, native, aus Pflanzenfasern gewonnene Cellulose enthalten, wobei sich die Gewichtsprozente auf das trockene Primärpartikelgesamtgewicht beziehen, und
B) mindestens einen Binder.

Ein Vorteil der vorliegenden Erfindung ist es, dass die erfindungsgemäßen Partikel Wirkstoffe aller Art in hohen Mengen zu absorbieren vermögen.
Ein weiterer Vorteil der vorliegenden Erfindung ist es, dass die erfindungsgemäßen Partikel im Wässrigen die absorbierten Substanzen in hoher Menge freisetzen.
Ein weiterer Vorteil der vorliegenden Erfindung ist es, dass die erfindungsgemäßen Partikel im Wässrigen die absorbierten Substanzen sehr schnell freisetzen.
Noch ein Vorteil der vorliegenden Erfindung ist es, dass die erfindungsgemäßen Partikel eine hervorragende Fließfähigkeit aufweisen.
Ein weiterer Vorteil der vorliegenden Erfindung ist es, dass die erfindungsgemäßen Partikel mechanisch stabil sind.
Ein weiterer Vorteil der vorliegenden Erfindung ist es, dass die erfindungsgemäßen Partikel komplett auf nachwachsenden Rohstoffen basierend hergestellt werden können.
Noch ein Vorteil der vorliegenden Erfindung ist es, dass die erfindungsgemäßen Partikel bioabbaubar sind.
Ein weiterer Vorteil der vorliegenden Erfindung ist es, dass die erfindungsgemäßen Partikel sich sehr gut tablettieren lassen, wobei insbesondere wenige Zusatzstoffe eingesetzt werden müssen. Noch ein Vorteil der vorliegenden Erfindung ist es, dass die mit Hilfe der erfindungsgemäßen Partikel hergestellten Tabletten eine hohe Bruchfestigkeit aufweisen. Noch ein Vorteil der vorliegenden Erfindung ist es, dass die mit Hilfe der erfindungsgemäßen Partikel hergestellten Tabletten eine geringe Masse aufweisen.
Gegenstand der vorliegenden Erfindung sind somit feste Partikel mit einer mittleren Partikelgröße von 30 $\mu$m bis 2000 $\mu$m, bevorzugt von 50 $\mu$m bis 200 $\mu$m, besonders bevorzugt von 120 $\mu$m bis 180 $\mu$m, enthaltend

A) feste Primärpartikel mit einer mittleren Partikelgröße von 3 $\mu$m bis 20 $\mu$m, bevorzugt von 8 $\mu$m bis 15 $\mu$m, besonders bevorzugt von 9 $\mu$m bis 12 $\mu$m, welche zu mindestens 95 Gew.-%, bevorzugt mindestens 97 Gew.-%, besonders bevorzugt mindestens 99 Gew.-% native, aus Pflanzenfasern gewonnene Cellulose enthalten, wobei sich die Gewichtsprozente auf das trockene Primärpartikelgesamtgewicht beziehen, und
B) mindestens einen Binder.

Unter dem Begriff "native, aus Pflanzenfasern gewonnene Cellulose" ist im Zusammenhang mit der vorliegenden Erfindung eine Cellulose zu verstehen, die keine chemische Modifikation in Form einer konzentrierten Säure- oder Basebehandlung erfahren hat, durch die die amorphen Anteile der Cellulose mindestens teilweise entfernt werden, und insbesondere keine chemische Derivatisierung wie z.B. Hydroxypropylierung, Hydroxyethylierung, Carboxymethylierung, Veresterung (z.B. Acetylierung), Veretherung (z.B. Methylierung) und Quaternierung erfahren hat, sondern lediglich aus einem Naturstoff über Vermahlen im Wässrigen erhalten wurde.

Unter dem Begriff "trocken" ist im Zusammenhang mit der vorliegenden Erfindung eine Cellulose beschrieben, die folgende Trocknung durchlaufen hat:

10 g der Cellulose werden bei 105 °C 2 Stunden im Trockenschrank gelagert.

Die Restfeuchte beträgt vor der Trocknung maximal 9 %.

Nach Abkühlung auf Raumtemperatur im Exsikkator wird die Probe ausgewogen.

Berechnung:

100 % - ((Gewicht nach Trocknung (g) * 100 %)/Einwaage vor Trocknung (g))= (%) Restfeuchte Unter dem Begriff "fest" ist im Zusammenhang mit der vorliegenden Erfindung der Aggregatzustand "fest" bei einer Umgebungstemperatur, bei der kosmetische Formulierungen eingesetzt werden, zu verstehen, diese Temperaturbereich erstreckt sich insbesondere von 15 °C bis 45 °C.

Alle Bedingungen wie beispielsweise Druck und Temperatur sind, wenn nicht anders angegeben, Standardbedingungen (25 °C, 1 bar). Prozentzahlen sind, soweit nicht anders beschrieben, in Massenprozent angegeben.

Die mittlere Partikelgröße wurde mit Hilfe der Laserbeugungs-Partikelgrößenanalyse im Horiba LA 950 von Retsch GmbH, Deutschland, bestimmt. Bei der Wechselwirkung von Laserlicht mit Partikeln werden durch Beugung, Brechung, Reflexion und Absorption für die Partikelgröße charakteristische Streulichtmuster erzeugt. Diese Streulichtmuster werden mit der Fraunhofer-Theorie einer bestimmten Korngrößenverteilung zugeordnet und die mittlere Partikelgröße stellt den d50 Wert bei der volumengewichteten Partikelgrößenverteilung dar. Das Gerät kann Partikel im Größenbereich zwischen 0,1 - 3.000 $\mu$m analysieren.

Das Cellulosepulver wurden trocken gemessen. Dabei wurden folgende Einstellungen am Gerät gewählt:

- Druckluft: 0.40 MPa
- Rinne: Auto
- Art der Verteilung: Volumen
- Brechungsindex (R): Fraunhofer RT[FH RT(2.000 - 5.600i)]

Die Stabilität der Pulver wird über den Abrieb während eines Siebvorgangs bestimmt. Für den Test wurden 5-10 g Pulver auf ein Sieb mit 63 $\mu$m Maschenweite gegeben und auf dem Siebturm abgesiebt (10 min, 2,5 mm Amplitude).

Der Gehalt an Cellulose in den Primärpartikeln wird folgendermaßen bestimmt:

2,5 g zerkleinerte Probe (Trockengehalt in einer gesonderten Probe ermitteln: 100% - TV % (TV: Trocknungsverlust)) werden in ein 150 ml Becherglas eingewogen (E). Mittels Pipette werden 30 ml auf 20 °C temperierte 17,5%-ige Natronlauge zur Probe gegeben. Die Masse wird mit einem Glasstab, dessen eines Ende abgeplattet ist, vorsichtig zerdrückt und 30 Minuten stehengelassen. Nach dieser Zeit wird mit 100 ml RO (*reverse osmosis*)-Wasser schnell verdünnt, sofort umgerührt und abgesaugt. Die zu verwendende Glasfritte G3 ist vorher im Trockenschrank zu trocknen, im Exsikkator abzukühlen und zu wiegen ($B_{leer}$). Es ist streng darauf zu achten, dass die verdünnte Masse so rasch wie möglich von der alkalischen Flüssigkeit abgesaugt und auch das anschließende Waschen zügig durchgeführt wird. Das Auswaschen erfolgt mit RO-Wasser portionsweise. Es wird solange gewaschen, bis gegenüber pH-Papier keine alkalische Reaktion mehr eintritt. Hierauf wird der Filterkuchen mit 0,5%-iger Salzsäure übergossen und 10 Minuten ohne Absaugen stehengelassen. Dann wird wieder mit RO-Wasser gewaschen, bis das pH-Papier keine Säurereaktion mehr zeigt. Die Durchführung findet bei 20 °C statt. Danach wird die Fritte bei 105 °C bis zur Gewichtskonstanz getrocknet, im Exsikkator abgekühlt und ausgewogen ($B_{Ausw}$).

$$\text{Gehalt Cellulose (\%)} = \frac{(B_{Ausw} - B_{leer}) \times 100 \times 100}{E \times (100 - TV)}$$

Grundlegender Unterschied von mikrokristallinen Cellulosen zu der hier eingesetzten Cellulose ist die Eigenschaft, dass die Primärpartikel schwer wasserlöslich sind und als feste Partikel vorliegen. Daher sind erfindungsgemäß insbesondere Partikel bevorzugt, die dadurch gekennzeichnet sind, dass die enthaltenen Primärpartikel eine maximale Löslichkeit in Wasser bei pH 7,0, 20 °C, 1 bar, von 0 g/L bis 0,5 g/L, bevorzugt von 0 g/L bis 0,2 g/L, besonders bevorzugt von 0 g/L bis 0,08 g/L aufweisen.

Erfindungsgemäß bevorzugte Partikel sind dadurch gekennzeichnet, dass sie eine Schüttdichte von 100-300 g/L, bevorzugt 120-270 g/L, besonders bevorzugt 140-240 g/L aufweisen. Die Schüttdichte wird bestimmt nach DIN 53468.

Erfindungsgemäß bevorzugte Partikel sind dadurch gekennzeichnet, dass sie ein Ölabsorptionsvermögen von 1,3 g bis 1,6 g Limonen pro g trockenem Partikel aufweisen. Bestimmt wird das Öl-/Wasserabsorptionsvermögen wie in den Beispielen beschrieben.

Die in den erfindungsgemäßen Partikeln enthaltenen Primärpartikeln enthalten bevorzugt native, aus Pflanzenfasern gewonnene Cellulose aufweisend einen Kristallinitätsgrad von 40 bis 90 %, bevorzugt von 50 bis 85%, besonders bevorzugt von 60 bis 80 %.

Für die quantitative Bestimmung der Kristallinität von Cellulose-Proben wird folgende "peak height"-Methode verwendet, beschrieben zum Beispiel bei. N. Terinte, R. Ibbett und K. C. Schuster, Lenzinger Berichte 89 (2011) 118-131:

Es werden Röntgenbeugungsaufnahmen im Bereich von 5°-45° (2 Θ) in Reflexion erstellt.

Die Luftstreuungskurve wird mittels des rein-kristallinen Standards NIST640c bestimmt, und diese als Untergrund für die Röntgenbeugungs-Diagramme der Messproben benutzt. Dieser Untergrund wird von der Messprobe abgezogen.

Der Kristallinitätsgrad CI wird als Verhältnis der Peakhöhe von dem kristallinen Signal I(002) bei 22° (2 Θ) nach dem Abziehen des nicht-kristallinen Beitrags I(nicht-kristallin) (das Signal bei 18° (2 Θ) und der Peakhöhe des kristallinen Peaks I(002) bei 22° (2 Θ) berechnet:

$$CI=((I(002)-I(nicht\text{-}kristallin))/I(002))*100 \text{ \%}$$

Die in den erfindungsgemäßen Partikeln enthaltenen Primärpartikeln enthalten bevorzugt native, aus Pflanzenfasern gewonnene Cellulose aufweisend einen mittleren Polymerisierungsgrad von 1 bis 50000, bevorzugt von 50 bis 20000, besonders bevorzugt von 200 bis 3000, aufweist.

Der mittlere Polymerisierungsgrad wird über die Messung der relativen Viskosität der in einer Cuen (Kupfer(II)ethylendiamin)-Lösung gelösten Cellulose wie folgt ermittelt.

Einwaage von 1,3 g Probe in einen 100 ml Erlenmeyerkolben ein (WS). Der Trockengehalt der Probe (TV) bzw. die Restfeuchte ist separat zu bestimmen.

Nachspülen mit 25 ml RO-Wasser, das vor der Verwendung mit Stickstoff gespült ist, und Verteilen der Cellulose im Wasser durch Schwenken.

Zufügen von 25 ml 1M Cuen-Lösung, die vor der Verwendung mit Stickstoff gespült ist.

Einleiten von Stickstoff in die Probelösung. Verschließen des Erlenmeyerkolbens mit dem dazugehörigen Glasstopfen. Schütteln der Probe, bis sich die Cellulose vollständig aufgelöst hat. Überführung von ca. 15 ml dieser Lösung in ein Ubbelohde Viskosimeter mit der Kapillare 1c. Temperieren der Probelösung auf 25°C ± 0,1°C. Mit einer Pipetteierhilfe wird die Lösung durch die Viskosimeterkapillare bis über die obere Glaskugel gezogen. Stoppen der Zeit, die die Lösung benötigt, um von der oberen zur unteren Markierung zu fließen. Wiederholung des Vorgangs und Berechnung aus beiden Zeitnahmen des Mittelwerts t1, falls beide Werte nicht mehr als 1% voneinander abweichen. Ansonsten ist eine weitere Bestimmung durchzuführen, der Mittelwert zweier nicht mehr als 1% voneinander abweichenden Ergebnisse ist zu bestimmen. Wiederholung des Vorgangs ohne Cellulose zur Bestimmung des Blindwerts. Hierzu wird die Kapillare 1 verwendet. Der erhaltene Mittelwert t2 ist die Fließzeit der reinen Cuen-Lösung.

Berechnung der relativen Viskosität:

$$\eta rel=(t1*k1)/(t2*k2)$$

$t_1 =$     Fließzeit der Probelösung (Mittelwert)

$t_2 =$     Fließzeit der Cuen-Lösung (Mittelwert)

$k_1 =$     Konstante des Ubbelohde Viskosimeters, Kapillare 1c

$k_2 =$     Konstante des Ubbelohde Viskosimeters, Kapillare 1

Aus der Tabelle 0315.-1. in Ph. Eur. 6.3. Powdered Cellulose wird der Wert für die intrinsische Viskosität zur relativen Viskosität für die Lösung der Cellulose entnommen. Der Polymerisierungsgrad wird als

$$PG=(9500*\eta c)/E*(100\text{-}TV)$$

berechnet, wobei

$\eta$ c: intrinsische Viskosität, E: Einwaage, TV: Trocknungsverlust in % ist.

Erfindungsgemäß bevorzugte Partikel sind dadurch gekennzeichnet, dass die in den Primärpartikel enthaltene native, aus Pflanzenfasern gewonnene Cellulose einen Gehalt von Cellulose Typ I im kristallinen Anteil größer als 95 Gew.-%,

besonders bevorzugt größer als 99 Gew.-% bezogen auf die Gesamtkristallinität aufweist.

Die verschiedenen Cellulosetypen sind zum Beispiel beschrieben bei Park et al. Biotechnology for Biofuels 2010, 3:10. Die Bestimmung des Cellulose-Typs wurde aufgrund eines Abgleichs der Röntgenbeugungsdiagramme mit den in der ICSD-Datenbank (Inorganic Crystal Structure Database) vorhandenen Referenzdiagrammen durchgeführt. Dieser Abgleich erfolgte aufgrund der Peaklagen und Intensitätsverhältnissen mit Hilfe der Softwaresuchfunktion von HighScore Plus (Hersteller: PANalytical) in der Version: 3.0c.

Erfindungsgemäß bevorzugte Partikel sind dadurch gekennzeichnet, dass sie sprühgetrocknete Partikel sind und eine mittlere Partikelgröße von 120 $\mu$m bis 180 $\mu$m aufweisen und die enthaltenen Primärpartikel eine mittlere Partikelgröße von 3 $\mu$m bis 15 $\mu$m aufweisen.

Erfindungsgemäß bevorzugte Partikel sind dadurch gekennzeichnet, dass sie die festen Primärpartikel enthaltend native, aus Pflanzenfasern gewonnene Cellulose in einer Menge von 60 Gew.-% bis 95 Gew.-%, bevorzugt 70 Gew.-% bis 90 Gew.-%, besonders bevorzugt 75 Gew.-% bis 85 Gew.-%, enthalten.

Erfindungsgemäß bevorzugte Partikel sind dadurch gekennzeichnet, dass der Binder ausgewählt ist aus der Gruppe umfassend, bevorzugt bestehend aus, Guar, Alginsäure, Alginat, Dextrin, Carbomer, Maltodextrin, Methylcellulose, Ethylcellulose, Gummi Arabicum, Hydroxyethylcellulose, Hydroxypropylcellulose, Hydroxypropylmethylcellulose, Hydroxyethylmethylcellulose, Carboxymethylcellulose, Baumwollsamenöl, Povidon, Ceratonia, Dextrose, Polydextrose, Stärke, Gelatine, vorgelierte Stärke, hydriertes Pflanzenöl, Maltodextrin, mikrokristalline Cellulose, Polyethylenoxid, Polymethacrylate, Cellulosefasern, bevorzugt Gummi Arabicum (1-10%) Carboxymethylcellulose (8-10%) Methylcellulose (1-30%), Cellulosefasern (5-15%), Ethylcellulose (1-10%) und Hydroxypropylmethylcellulose (1-10%). Die Werte in Klammern geben bevorzugte Gewichtsbereiche bezogen auf das Gesamtpartikelgewicht des jeweiligen Binders an.

Es ist erfindungsgemäß bevorzugt, dass die Partikel kein Epoxidharz enthalten.

Erfindungsgemäß bevorzugte Partikel sind dadurch gekennzeichnet, dass sie den Binder in einer Menge von 1 Gew.-% bis 30 Gew.-%, bevorzugt 2 Gew.-% bis 20 Gew.-%, besonders bevorzugt 5 Gew.-% bis 10 Gew.-%, enthalten.

Ein weiterer Gegenstand der vorliegenden Erfindung ist ein Verfahren zur Herstellung von festen Partikeln mit einer mittleren Partikelgröße von 15 $\mu$m bis 2000 $\mu$m, bevorzugt von 50 $\mu$m bis 200 $\mu$m, besonders bevorzugt von 120 $\mu$m bis 180 $\mu$m, umfassend die Verfahrensschritte

A) Bereitstellen feste Primärpartikel mit einer mittleren Partikelgröße von 3 $\mu$m bis 20 $\mu$m, bevorzugt von 8 $\mu$m bis 15 $\mu$m, besonders bevorzugt von 9 $\mu$m bis 12 $\mu$m, welche zu mindestens 95 Gew.-%, bevorzugt mindestens 97 Gew.-%, besonders bevorzugt mindestens 99 Gew.-% native, aus Pflanzenfasern gewonnene Cellulose enthalten, wobei sich die Gewichtsprozente auf das trockene Primärpartikelgesamtgewicht beziehen,

B) gegebenenfalls Versetzen mit Flüssigkeit und/oder einem Binder,

C) Agglomerieren der Primärpartikeln durch Aufgranulieren, Kompaktieren oder Sprühtrocken, insbesondere Sprühtrocken.

Das erfindungsgemäße Verfahren setzt bevorzugt solche Binde ein, die in den erfindusngemäßen Partikeln bevorzugt enthalten sind. Analoges gilt für die Primärpartikel.

*Sprühtrocknung:*

Das erfindungsgemäße Verfahren zur Herstellung von festen Partikeln mit einer mittleren Partikelgröße von 15 $\mu$m bis 250 $\mu$m, bevorzugt von 50 $\mu$m bis 200 $\mu$m, besonders bevorzugt von 120 $\mu$m bis 180 $\mu$m, ist bevorzugtes dadurch gekennzeichnet, dass in Verfahrensschritt B) mit Flüssigkeit und bevorzugt mit einem Binder versetzt wird und das Agglomerieren in Verfahrensschritt C) durch Sprühtrocknen erfolgt.

Es ist erfindungsgemäß bevorzugt, dass der Binder in der Flüssigkeit gelöst vorliegt. Bevorzugt werden die Primärpartikel in einer Flüssigkeit dispergiert, insbesondere mit Hilfe einer intensiven Rotor-Stator Maschine (z.B. IKA Ultra-Turrax). Bevorzugt erfolgt Verfahrensschritt B) in einem Rührbehälter, insbesondere unter Homogenisierung der Flüssigkeit, gegebenenfalls des Binders und der Primärpartikel. Bevorzugt beträgt die Feststoffkonzentration, bezogen auf die Flüssigkeit, gegebenenfalls den Binder und die Primärpartikel 5 Gew.-% bis 30 Gew.-%, bevorzugt 10 Gew.-% bis 30 Gew.-%, besonders bevorzugt 15 Gew.-% bis 20 Gew.-%.

Verfahrensschritt C) wird bevorzugt in einem Sprühtrocknungsturm durchgeführt, in dem die Flüssigkeit, gegebenenfalls der Binder und die Primärpartikel zerstäubt werden bevorzugt unter Einsatz einer Zweistoffdüse, Druckdüse oder eines Zentrifugalzerstäubers.

Die Sprühtrocknung kann mit einem Trocknungsgas, insbesondere Stickstoff im Gleichstrom oder im Gegenstrom durchgeführt werden. Bevorzugt wird das Trocknungsgas von den festen Partikeln mit Hilfe eines Zyklons getrennt.

*Aufgranulieren:*

Das erfindungsgemäße Verfahren zur Herstellung von festen Partikeln mit einer mittleren Partikelgröße von 200 $\mu$m bis 450 $\mu$m ist bevorzugt dadurch gekennzeichnet, dass das Agglomerieren in Verfahrensschritt C) durch Aufgranulieren erfolgt.

Das erfindungsgemäße Verfahren ist in diesem Zusammenhang bevorzugt dadurch gekennzeichnet, dass in Verfahrensschritt B) der Binder in der Flüssigkeit gelöst zu den Primärpartikeln eingetropft oder über eine Düse zugeführt wird.

Des Weiteren ist dieses Verfahren bevorzugt dadurch gekennzeichnet, dass es den Verfahrensschritt D) Trocknen der Partikel, insbesondere in einem Umluftofen, bevorzugt in einem Temperaturbereich von 60 °C bis 140 °C, bevorzugt von 80 °C bis 120 °C, besonders bevorzugt von 90 °C bis 110 °C, umfasst.

Gegebenenfalls erfolgt eine Größenfraktionierung der festen Partikeln zu einer mittleren Partikelgröße von 200 $\mu$m bis 450 $\mu$m, insbesondere durch Sieben.

*Kompaktieren:*

Das erfindungsgemäße Verfahren zur Herstellung von festen Partikeln mit einer mittleren Partikelgröße von 200 $\mu$m bis 450 $\mu$m ist bevorzugt dadurch gekennzeichnet, dass das Agglomerieren in Verfahrensschritt C) durch Kompaktieren erfolgt.

Es ist in diesem Zusammenhang erfindungsgemäß bevorzugt, dass das Kompaktieren in Verfahrensschritt C) mit einem Walzenkompaktator durchgeführt wird und bevorzugt das erfindungsgemäße Verfahren den Verfahrensschritt E) Zerkleinern der Kompaktate aus Verfahrensschritt C), bevorzugt mit einem Passiersieb, und Größenfraktionierung der festen Partikeln zu einer mittleren Partikelgröße von 200 $\mu$m bis 2000 $\mu$m, insbesondere durch Sieben, umfasst.

Bevorzugt werden die Primärpartikel und gegebenenfalls der Binder mit einer Stopfschnecke dem Walzenkompaktator zugeführt. Es ist in diesem Zusammenhang erfindungsgemäß bevorzugt, dass kein Binder eingesetzt wird. Der Walzenkompaktator ist bevorzugt ein Bepex L200/50 G+K (Hutt 2).

Ein weiterer Gegenstand der vorliegenden Erfindung sind die Partikel erhältlich nach dem erfindungsgemäßen Verfahren.

Noch ein weiterer Gegenstand der vorliegenden Erfindung ist die Verwendung eines erfindungsgemäßen Partikels zur Absorption, bevorzugt mit folgender Desorption in wässriger Umgebung, mindestens einer Substanz ausgewählt aus der Gruppe Aromastoffe, kosmetische und pharmazeutische Wirkstoffe.

Bei dieser erfindungsgemäßen Verwendung werden insbesondere bevorzugte feste Partikel verwendet, die oben bevorzugt vorbeschrieben sind.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

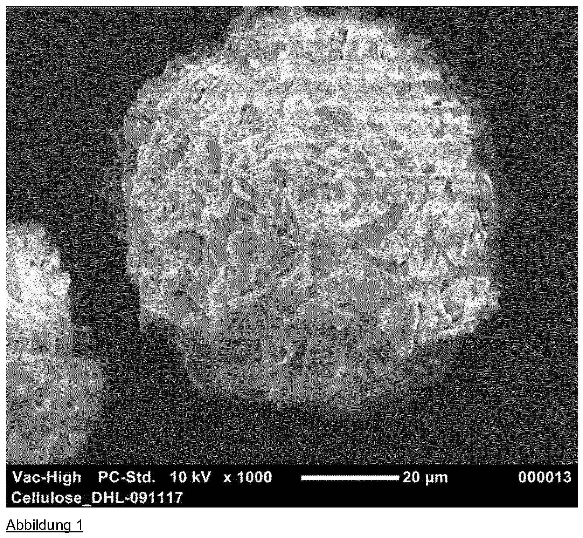

Abbildung 1: Sprühgetrockneter Partikel enthaltend feste Primärpartikel mit einer mittleren Partikelgröße von 9 $\mu$m (erfindungsgemäß)

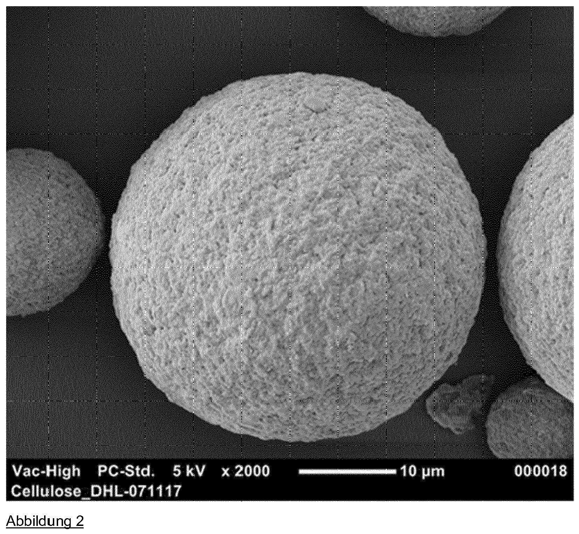

Abbildung 2: Sprühgetrockneter Partikel enthaltend feste Primärpartikel mit einer mittleren Partikelgröße von 2 $\mu$m (nicht erfindungsgemäß)

Beispiele:

*Beispiel 1: Ölabsorption und Fließfähigkeit*

Es wurden auf Basis kommerziell erhältlicher nativer, aus Pflanzenfasern gewonnener Cellulose sprühgetrocknete Partikel wie folgt hergestellt:

Sprühtrocknung:

Die Suspension von den Cellulose-Primärpartikeln in Wasser (5-25 % Gew. Cellulose) wurde mit Hilfe eines Dispergierers hergestellt und gegebenenfalls mit einer Binderlösung für 30 Minuten in mit einem Überkopfrührer gemischt. Diese Dispersion wurde anschließend mit Hilfe einer Schlauchpumpe zum Sprühtrockner (Niro Minor von GEA®) gefördert (2,5 kg/Std) und mit einer Zweistoffdüse zerstäubt (Zerstäubungsgas: Stickstoff, 0.5 bar). Getrocknet wurde mit heißem Stickstoff (15 kg/h, $T_{ein}$= 240°C, Gleichstrom). Die Partikeln wurden im Zyklon geerntet.

Falls Binder (i.e. Methylcellulose) eingesetzt wurde, wurde diese in Form einer wässrigen Zusammensetzung zuerst präpariert: Pulverförmige Methylcellulose (MC) wurde unter Rühren zu derselben Wassermenge, wie in der zu verinigenden Cellulose-Dispersion enthalten ist, bei einer Temperatur von 80°C hinzugefugt. Nach 20 Minuten, sobald sich die Methylcellulose fein verteilt hatte, wurde ein noch einmal dieselbe Wassermenge, welches eine Temperatur von 20°C aufwies, hinzugefügt, und die Zusammensetzung bis auf 0-5°C unter Rühren gekühlt. Es folgte Rühren für weitere 40 Minuten, bis die Methylcellulose vollständig gelöst war.

Im erfindungsgemäßen Größenbereich der Primärpartikel wurde Tego®Feel Green sowie Diacel 10 eingesetzt; unterhalb des erfindungsgemäßen Bereiches diente fein vermahlenes Tego®Feel Green, oberhalb des beanspruchten Bereiches Tego® C10 als Primärpartikel.

In Produkt 5 wurde als zusätzlicher Binder Methylcellulose beigefügt.

Bestimmung der Öladsorption

Vortrocknen des Trägermaterials: 1,5 g des Trägerstoffes (Cellulose) in eine 100 ml Laborgewindeflasche einwiegen und offen über Nacht im Vakuumtrockenschrank trocknen. (45 °C, 20 mbar). Wenn nötig die Öffnung mit einem Papiertuch und einem Gummiband verschließen, um Materialverlust beim Einschalten des Vakuums zu verhindern Beladen des Trägermaterials: Die getrockneten Proben anschließend mit der 3g Öl (Limonen) beladen und mit einem Spatel gut durchmischen. Möglichst darauf achten, dass nicht zu viel der Mischung am Spatel kleben bleibt. Die Gewindeflasche mit dem Deckel verschließen und für ca. 3 Stunden stehen lassen.

Zentrifugation: 5 Rundfiltern ($\varnothing$ 5,5 cm) trichterförmig falten und in ein 50ml Falcon Tube einsetzen. 3 g der Cellulose-Öl-Mischung in das Falcon Tube einwiegen und darauf achten, dass die Mischung nicht seitlich am Filter vorbeilaufen kann oder an den Wänden des Falcon Tubes haftet Das befüllte Falcon Tube zentrifugieren (Hettich Zentrifugen Rotina 380R - radius Rotor: 14,8 cm / RPM: 4300 / Dauer: 6 min)

Auswiegen des Filterkuchens: Leergewicht einer geeigneten Glasschale bestimmen (möglichst klein, da zu große Schalen zu ungenauen Gewichtsmessungen auf der Analysewaage führen). Den gesamten Filterkuchen in die Glasschale geben, mit dem Spatel etwas zerkleinern und wiegen Trocknen des Filterkuchens: Anschließend die befüllte Glasschale mindestens 12 h im Vakuumtrockenschrank trocknen (45°C, 20 mbar) und dann erneut wiegen (Die Glasschale wieder mit einem Papiertuch und einem Gummiband abdecken). Aus der Gewichtsdifferenz vor und nach dem Trocknen die Beladung des Trägers mit Öl vor der Trocknung bestimmen

Bestimmung der Fließfähigkeit

Die Fließfähigkeit der Trägerstoffe wurde mit einer Reihe von Glastrichtern mit verschiedenen Austrittsöffnungen bestimmt. Für den Test werden die Trichter mit Hilfe einer Halterung oberhalb eines Auffanggefäßes fixiert. Zur Abdeckung der Trichteröffnung wird eine Spielkarte zwischen Trichter und Gefäß geklemmt. Der Trichter wird bis zwei cm unterhalb der Oberkante des Trichters mit dem Trägerstoff befüllt. Anschließend wird die Karte entfernt und das Auslaufen des Pulvers nach folgender Skala beurteilt.

| Bewertung | Auslauf Trichter mit d [mm]* | Fließfähigkeit |
|---|---|---|
| 1 | 2,5 | sehr gut |
| 2 | 5,0 | |
| 3 | 8,0 | |
| 4 | 12,0 | |
| 5 | 18,0 | |
| 6 | 24,0 | Schlecht |
| 7 | - | kein Auslauf aus 6 |
| * Probe läuft mit einmal Klopfen flüssig aus; falls erst mit 2-4 mal Klopfen, dann Bewertung +0,5 | | |

Tabelle 1: Cellulose Primärpartikel Eigenschaften. "-" nicht bestimmt; "/" Messung nicht möglich

| Primärpartikel | d50 der Primärpartikel in $\mu$m | Fließfähigkeit der Primärpartikel | Ölabsorptionsvermögen g Öl/g Primärpartikel | Stabilität der Primärpartikel; Feinanteil Abrieb in % | Schüttdichte in g/L |
|---|---|---|---|---|---|
| 1: Tego®Feel Green | 8,75 | 7 | 1,2 | 93 | 183 |
| 2: Diacel 10 | 9,0 | 7 | 1,1 | - | 200 |
| 3: Zermahlenes Tego®Feel Green | 3 | / (wässerige Suspension) | / (wässerige Suspension) | / (wässerige Suspension) | / (wässerige Suspension) |
| 4: Tego® C10 | 44 | 7 | 1,2 | - | 252 |
| 6: Microcrystalline cellulose | 150 | 7 | 0,4 | 49 | 345 |

Tabelle 2: Ergebnisse der Herstellung von Cellulose Partikeln. "-" nicht bestimmt; "/" Messung nicht möglich; "*" erfindungsgemäß

| Sprühgetrocknete Produkt | d50 Partikel in $\mu$m | Ölabsorptionsvermögen g Öl/g Partikel | Stabilität der Partikel; Feinanteil Abrieb in % | Schüttdichte in g/L |
|---|---|---|---|---|
| 1*: Tego®Feel Green | 50 | 1,31 | 63 % | 242 |
| 2*: Diacel 10 | 66 | 1,2 | - | 253 |
| 3: Zermahlenes Tego®Feel Green | 18 | 0,7 | / | 596 |
| 4: Tego® C10 | Keine Partikel geformt | | | |
| 5*: Tego®Feel Green mit, 10% Methylcellulose | 62 | 1,37 | 43 % | 235 |

Aus den in der Tabelle gelisteten Ergebnissen ist unmittelbar ersichtlich, dass die Sprühtrocknung der im erfindungsgemäßen Größenbereich liegenden Primärpartikeln zu Partikeln mit erhöhtem Ölabsorptionsvermögen führt. Sind die Primärpartikel zu klein, ist das Ölabsorptionsvermögen gering, sind sie zu groß, werden keine fließfähigen Partikel erhalten.
Das Beifügen von Bindern wie Methylcellulose erhöht die mechanische Stabilität der Partikel, gekennzeichnet durch verringerte Entstehung von Feinanteil.

*Beispiel 2: Absorption und Desorption von pharmazeutischen Wirkstoffen.*

4-[5-(4-Methylphenyl)-3-(trifluormethyl)-pyrazol-1-yl]benzensulfonamid (Celecoxib, Aarti Drugs Ltd., Mumbai, Indien) wurde auf verschiedene Cellulose Präparate geladen.
Die Desorption wurde mit 25 mg oder einer äquivalenten Menge Celecoxib in einem USP apparatus II (Pharma Test PWTS 1210) in 500 ml 0.1N HCl bei 100 rpm und 37 $\pm$ 0,5 °C untersucht.
Der Wirkstoff Celecoxib wurde in einer Mischung verschiedener Komponenten inkorporiert, welche aus Miglyol® 812, Tween® 80, Gelucire® 44/14 und D-$\alpha$-Tocopherol polyethylene glycol 1000 succinate (d-TPGS) besteht.
Die Cellulose Präparate werden mit der letztgenannten öligen Formulierung, welche Celecoxib enthält beladen.

Tabelle 3: Ergebnisse der Herstellung von Cellulose Partikeln. "*" erfindungsgemäß

| | d50 Partikel in μm | Beladung g/g Partikel | Fließfähigkeit 1 - 7 | Release % 5 min | Release % 10 min | Release % 15 min |
|---|---|---|---|---|---|---|
| **1\*:** Diacel 10 Cellulose | 14,4 | 1,15 | 5 | 82 | 91 | 95 |
| **2\*:** Diacel 10 Cellulose + 10% MC | 16,3 | 1,23 | 3 | 80 | 88 | 95 |
| **3\*:** Diacel 10 Cellulose + 20% MC | 17,5 | 1,26 | 3 | 88 | 98 | 100 |
| **4\*:** Diacel 10 Cellulose + 30% MC | 18,7 | 1,26 | 3 | 80 | 93 | 96 |
| **5\*:** Tego®Feel Green Cellulose + 6% Stärkekleber | 300 | 1,29 | 3 | 83 | 89 | 90 |
| **6:** Avicel PH-101 | 50 | 0,90 | 7 | 83 | 86 | 89 |
| **7:** Aeroperl® 300 Pharma | 30 | 1,40 | 2 | 23 | 30 | 32 |
| **8:** Syloid® XDP 3050 | 50 | 1,44 | 2 | 17 | 21 | 22 |

Aus den in der Tabelle gelisteten Ergebnissen ist unmittelbar ersichtlich, dass die erfindungsgemäßen Partikel sehr hohe Beladungsraten erreichen, und diese sehr hohen Beladungsraten überdies hinaus auch zur schnelleren Freisetzung von pharmazeutischen Wirkstoffen führen, als dies für herkömmliche Partikel der Fall ist. Im Vergleich zu Siliciumdoxidbasierten Adsorbentien (siehe Tabelle 3) setzen die erfindungsgemäßen Partikel den Wirkstoff ebenfalls schneller und in größerem Ausmaß frei. Zudem weisen die erfindungsgemäßen Partikel eine bessere Fließfähigkeit als die herkömmliche mikrokristalline Cellulose (Avicel PH-101) auf, zeigen zu den Siliciumdioxid basierten Adsorbentien eine vergleichbar gute Fließfähigkeit und eignen sich auch zur Herstellung von Tabletten sowie zur Befüllung von Kapselgütern.

*Beispiel 3: Tablettierung*

Die erfindungsgemäßen Partikel wurden alleine und in Kombination mit anderen Komponenten zu Tabletten weiterverarbeitet. Für diesen Prozessschritt wurde die Tablettenpresse EP-1 (Exzenterpresse) vom Hersteller ERWEKA GmbH (Heusenstamm, Germany) verwendet. Für die Bestimmung der Dicke, des Durchmessers und der Bruchfestigkeit der verschiedenen Tabletten wurde der Tabletten-Bruchfestigkeitstester TBH 125 ebenfalls von der ERWEKA GmbH (Heusenstamm, Germany) herangezogen. Für die Bestimmung der genannten Tablettenparameter wurden für jede unterschiedliche Zusammensetzung jeweils 2 Tabletten (n = 2) analysiert.

Tabelle 3: Ergebnisse der Tablettierung von Cellulose Partikeln. "*" erfindungsgemäß

| | Dicke** [mm] | Durchmesser** [mm] | Bruchfestigkeit** [N] |
|---|---|---|---|
| **1\*:** Diacel 10 + Celecoxib (15%) | 4,34 ± 0,025 | 10,085 ± 0,005 | 89,5 ± 4,5 |
| **2: roh** Diacel 10 + Celecoxib (15%) | 4,90 ± 0,015 | 10,075 ± 0,015 | 77,5 ± 3,5 |
| **3:** Zermahlenes Tego®Feel Green + Celecoxib (15%) | 4,65 ± 0,040 | 10,065 ± 0,005 | 46,5 ± 3,5 |
| ** n = 2 ± S.D. | | | |

Die erfindungsgemäßen Partikel lassen sich mit Hilfe genannter Exzenterpresse zu Tabletten weiterverarbeiten. Die Verpressung der erfindungsgemäßen Partikel ist auch ohne den Zusatz weiterer Bestandteile möglich. Für die Vergleichbarkeit der Materialien untereinander wurden Tabletten mit einer Dicke im Bereich von ca. 4,3 -5,0 mm sowie eines Durchmesser von ca. 10,0 - 10,1 mm hergestellt.

Die Bruchfestigkeit der erhaltenen Tabletten (siehe Tabelle 3) liegt für aufgelistete Materialien im Bereich von ca. 30 - 142 N. Die Tabletten auf Basis der erfindungsgemäßen Partikel eignen sich durch die entsprechend ermittelte Bruchfestigkeit ebenfalls zur Weiterverarbeitung (z.B. Coating). Durch Zusatz des pharmazeutischen Wirkstoffs Celecoxib wird die Bruchfestigkeit der Tabletten sowohl derer auf Basis der erfindungsgemäßen Partikel als auch jener herkömmlicher Materialien erhöht. Wirkstoffbeladen konnten für Diacel 10 und Avicel PH-101 als Ausgangstoffe vergleichbare Bruchfestigkeiten ermittelt werden. Die Bruchfestigkeit für die Tabletten auf Basis von Tego® Feel Green liegt hierbei etwas höher.

**Patentansprüche**

1.  Feste Partikel mit einer mittleren Partikelgröße von 15 μm bis 2000 μm, bevorzugt von 50 μm bis 200 μm, besonders bevorzugt von 120 μm bis 180 μm, enthaltend

    A) feste Primärpartikel mit einer mittleren Partikelgröße von 3 μm bis 20 μm, bevorzugt von 8 μm bis 15 μm, besonders bevorzugt von 9 μm bis 12 μm, welche zu mindestens 95 Gew.-%, bevorzugt mindestens 97 Gew.-%, besonders bevorzugt mindestens 99 Gew.-% native, aus Pflanzenfasern gewonnene Cellulose enthalten, wobei sich die Gewichtsprozente auf das trockene Primärpartikelgesamtgewicht beziehen, und
    B) mindestens einen Binder.

2.  Partikel gemäß Anspruch 1, **dadurch gekennzeichnet, dass** sie sprühgetrocknete, aufgranulierte oder kompaktierte, insbesondere sprühgetrocknete, Partikel sind.

3.  Sprühgetrocknete Partikel gemäß Anspruch 1 mit einer mittleren Partikelgröße von 15 μm bis 250 μm, bevorzugt von 50 μm bis 200 μm, besonders bevorzugt von 120 μm bis 180 μm.

4.  Aufgranulatierte Partikel gemäß Anspruch 1 mit einer mittleren Partikelgröße von 200 μm bis 450 μm.

5.  Kompaktierte Partikel gemäß Anspruch 1 mit einer mittleren Partikelgröße von 200 μm bis 2000 μm.

6.  Partikel gemäß mindestens einem der vorherigen Ansprüche, **dadurch gekennzeichnet, dass** die enthaltenen Primärpartikel eine maximale Löslichkeit in Wasser bei pH 7,0, 20 °C, 1 bar, von 0 g/L bis 0,5 g/L, bevorzugt von 0 g/L bis 0,2 g/L, besonders bevorzugt von 0 g/L bis 0,08 g/L aufweisen.

7.  Partikel gemäß mindestens einem der vorherigen Ansprüche, **dadurch gekennzeichnet, dass** sie eine Schüttdichte von 100-300 g/L, bevorzugt 120-270 g/L, besonders bevorzugt 140-240 g/L aufweisen.

8.  Partikel gemäß mindestens einem der vorherigen Ansprüche, **dadurch gekennzeichnet, dass** die in den Primärpartikeln enthaltene native, aus Pflanzenfasern gewonnene Cellulose einen Kristallinitätsgrad von 40 bis 90 % aufweist.

9.  Partikel gemäß mindestens einem der vorherigen Ansprüche, **dadurch gekennzeichnet, dass** die in den Primärpartikel enthaltene native, aus Pflanzenfasern gewonnene Cellulose einen mittleren Polymerisierungsgrad von 1 bis 50000 aufweist.

10. Partikel gemäß mindestens einem der vorherigen Ansprüche, **dadurch gekennzeichnet, dass** die in den Primärpartikel enthaltene native, aus Pflanzenfasern gewonnene Cellulose einen Gehalt von Cellulose Typ I im kristallinen Anteil größer als 95 Gew.-%, besonders bevorzugt größer als 99 Gew.-% bezogen auf die Gesamtkristallinität aufweist.

11. Partikel gemäß mindestens einem der Ansprüche 1 bis 2 und 6 bis 10, **dadurch gekennzeichnet, dass** sie sprühgetrocknete Partikel sind und eine mittlere Partikelgröße von 120 μm bis 180 μm aufweisen und die enthaltenen Primärpartikel eine mittlere Partikelgröße von 3 μm bis 15 μm aufweisen.

12. Partikel gemäß mindestens einem der vorherigen Ansprüche, **dadurch gekennzeichnet, dass** der Binder ausgewählt ist aus der Gruppe umfassen, bevorzugt bestehend aus, Guar, Alginsäure, Alginat, Dextrin, Carbomer, Maltodextrin, Methylcellulose, Ethylcellulose, Gummi Arabicum, Hydroxyethylcellulose, Hydroxypropylcellulose, Hydroxypropylmethylcellulose, Hydroxyethylmethylcellulose, Carboxymethylcellulose, Baumwollsamenöl, Povidon, Ceratonia, Dextrose, Polydextrose, Stärke, Gelatine, vorgelierte Stärke, hydriertes Pflanzenöl, Maltodextrin, mikrokristalline Cellulose, Polyethylenoxid, Polymethacrylate, Cellulosefasern.

**13.** Verfahren zur Herstellung von festen Partikeln mit einer mittleren Partikelgröße von 15 $\mu$m bis 2000 $\mu$m, bevorzugt von 50 $\mu$m bis 200 $\mu$m, besonders bevorzugt von 120 $\mu$m bis 180 $\mu$m, umfassend die Verfahrensschritte

A) Bereitstellen feste Primärpartikel mit einer mittleren Partikelgröße von 3 $\mu$m bis 20 $\mu$m, bevorzugt von 8 $\mu$m bis 15 $\mu$m, besonders bevorzugt von 9 $\mu$m bis 12 $\mu$m, welche zu mindestens 95 Gew.-%, bevorzugt mindestens 97 Gew.-%, besonders bevorzugt mindestens 99 Gew.-% native, aus Pflanzenfasern gewonnene Cellulose enthalten, wobei sich die Gewichtsprozente auf das trockene Primärpartikelgesamtgewicht beziehen,
B) gegebenenfalls Versetzen mit Flüssigkeit und/oder einem Binder,
C) Agglomerieren der Primärpartikeln durch Aufgranulieren, Kompaktieren oder Sprühtrocknen, insbesondere Sprühtrocknen.

**14.** Verfahren zur Herstellung von festen Partikeln mit einer mittleren Partikelgröße von 15 $\mu$m bis 250 $\mu$m, bevorzugt von 50 $\mu$m bis 200 $\mu$m, besonders bevorzugt von 120 $\mu$m bis 180 $\mu$m, gemäß Anspruch 19, **dadurch gekennzeichnet, dass** in Verfahrensschritt B) mit Flüssigkeit und bevorzugt mit einem Binder versetzt wird und das Agglomerieren in Verfahrensschritt C) durch Sprühtrocknen erfolgt.

**15.** Verfahren zur Herstellung von festen Partikeln mit einer mittleren Partikelgröße von 200 $\mu$m bis 450 $\mu$m gemäß Anspruch 13, **dadurch gekennzeichnet, dass** das Agglomerieren in Verfahrensschritt C) durch Aufgranulieren erfolgt.

**16.** Verfahren gemäß Anspruch 15, **dadurch gekennzeichnet, dass** in Verfahrensschritt B) der Binder in der Flüssigkeit gelöst zu den Primärpartikeln eingetropft oder über eine Düse zugeführt wird.

**17.** Verfahren gemäß Anspruch 15 oder 16, **dadurch gekennzeichnet, dass** es den Verfahrensschritt
D) Trocknen der Partikel, insbesondere in einem Umluftofen, bevorzugt in einem Temperaturbereich von 60 °C bis 140 °C, bevorzugt von 80 °C bis 120 °C, besonders bevorzugt von 90 °C bis 110 °C,
umfasst.

**18.** Verfahren zur Herstellung von festen Partikeln mit einer mittleren Partikelgröße von 200 $\mu$m bis 2000 $\mu$m gemäß Anspruch 13, **dadurch gekennzeichnet, dass** das Agglomerieren in Verfahrensschritt C) durch Kompaktieren erfolgt.

**19.** Verfahren gemäß Anspruch 18, **dadurch gekennzeichnet, dass** das Kompaktieren in Verfahrensschritt C) mit einem Walzenkompaktator durchgeführt wird und bevorzugt es den Verfahrensschritt
E) Zerkleinern der Kompaktate aus Verfahrensschritt C), bevorzugt mit einem Passiersieb und Größenfraktionierung der festen Partikeln zu einer mittleren Partikelgröße von 200 $\mu$m bis 2000 $\mu$m, insbesondere durch Sieben, umfasst.

**20.** Partikel erhältlich nach einem Verfahren gemäß mindestens einem der Ansprüche 13 bis 19.

**21.** Verwendung eines Partikels gemäß mindestens einem der Ansprüche 1 bis 12 oder 20 zur Absorption, bevorzugt mit folgender Desorption in wässriger Umgebung, mindestens einer Substanz ausgewählt aus der Gruppe Aromastoffe, kosmetische und pharmazeutische Wirkstoffe.

Abbildung 1

Abbildung 2

Europäisches
Patentamt

European
Patent Office

Office européen
des brevets

# EUROPÄISCHER RECHERCHENBERICHT

Nummer der Anmeldung

EP 19 20 8352

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2010/055180 A1 (DEORKAR NANDU [US] ET AL) 4. März 2010 (2010-03-04) * Anspruch 1; Beispiele 1-39 * ----- | 1-21 | INV. A61K9/00 A61K9/14 A61K9/16 A61K47/38 |
| X | EP 3 459 523 A1 (JUJO PAPER CO LTD [JP]) 27. März 2019 (2019-03-27) * Absätze [0017], [1837], [0039], [0043], [0056]; Ansprüche 1-9; Beispiele 1-3 * ----- | 1-21 | |
| A | SHANGRAW R F ET AL: "Morphology and functionality in tablet excipients for direct compression: part I", PHARMACEUTICAL TECHNOLOGY, ADVANSTAR COMMUNICATIONS,INC, US, 1. Januar 1981 (1981-01-01), Seiten 69-78, XP008086153, ISSN: 1543-2521 * Seite 77, linke Spalte * ----- | 1-21 | |
| A | KIBBE A H (ED) ED - KIBBE A H (ED): "Cellulose, Powdered", 1. Januar 2000 (2000-01-01), HANDBOOK OF PHARMACEUTICAL EXCIPIENTS, AMERICAN PHARMACEUTICAL ASSOC. [U.A.], WASHINGTON, DC; US, PAGE(S) 102 - 105, XP002423964, ISBN: 978-0-85369-381-9 * das ganze Dokument * ----- | 1-21 | RECHERCHIERTE SACHGEBIETE (IPC) A61K |
| A | WO 95/17955 A1 (CULTOR OY [FI]) 6. Juli 1995 (1995-07-06) * Ansprüche 1,23; Beispiele 1-6 * ----- -/-- | 1-21 | |

Der vorliegende Recherchenbericht wurde für alle Patentansprüche erstellt

| Recherchenort | Abschlußdatum der Recherche | Prüfer |
|---|---|---|
| Den Haag | 1. April 2020 | Konter, Jörg |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
..............................................................
& : Mitglied der gleichen Patentfamilie, übereinstimmendes
    Dokument

EPO FORM 1503 03.82 (P04C03)

Seite 1 von 2

Europäisches
Patentamt

European
Patent Office

Office européen
des brevets

**EUROPÄISCHER RECHERCHENBERICHT**

Nummer der Anmeldung

EP 19 20 8352

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | SUHAS ET AL: "Cellulose: A review as natural, modified and activated carbon adsorbent", BIORESOURCE TECHNOLOGY, ELSEVIER, AMSTERDAM, NL, Bd. 216, 28. Mai 2016 (2016-05-28), Seiten 1066-1076, XP029631974, ISSN: 0960-8524, DOI: 10.1016/J.BIORTECH.2016.05.106 * Seite 1068 * ----- | 1-21 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 1. April 2020 | Konter, Jörg |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 20 8352

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-04-2020

| Im Recherchenbericht angeführtes Patentdokument | Datum der Veröffentlichung | Mitglied(er) der Patentfamilie | Datum der Veröffentlichung |
|---|---|---|---|
| US 2010055180 A1 | 04-03-2010 | US 2010055180 A1<br>US 2017112768 A1 | 04-03-2010<br>27-04-2017 |
| EP 3459523 A1 | 27-03-2019 | CN 109152721 A<br>EP 3459523 A1<br>JP 2018052909 A<br>JP WO2017199894 A1<br>KR 20190006534 A<br>US 2019099348 A1<br>WO 2017199894 A1 | 04-01-2019<br>27-03-2019<br>05-04-2018<br>09-05-2019<br>18-01-2019<br>04-04-2019<br>23-11-2017 |
| WO 9517955 A1 | 06-07-1995 | EP 0737098 A1<br>FI 962646 A<br>JP H09507787 A<br>US 5283123 A<br>WO 9517955 A1 | 16-10-1996<br>26-06-1996<br>12-08-1997<br>01-02-1994<br>06-07-1995 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• DE 2921931

**In der Beschreibung aufgeführte Nicht-Patentliteratur**

• **N. TERINTE ; R. IBBETT ; K. C. SCHUSTER.** *Lenzinger Berichte,* 2011, vol. 89, 118-131